(19) Europäisches Patentamt
European Patent Office
Office européen des brevets

(11) EP 4 564 003 A2

(12) # EUROPEAN PATENT APPLICATION

(43) Date of publication:
**04.06.2025 Bulletin 2025/23**

(21) Application number: **24216442.4**

(22) Date of filing: **29.11.2024**

(51) International Patent Classification (IPC):
***G01N 33/536*** *(2006.01)* ***G01N 33/544*** *(2006.01)*
***G01N 33/543*** *(2006.01)*

(52) Cooperative Patent Classification (CPC):
**G01N 33/536; G01N 33/54353; G01N 33/544**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **30.11.2023 JP 2023203380**

(71) Applicant: **CANON KABUSHIKI KAISHA**
**Tokyo 146-8501 (JP)**

(72) Inventors:
- **KAKU, Mai**
  **Tokyo (JP)**
- **NATORI, Ryo**
  **Tokyo (JP)**
- **KAWABE, Yudai**
  **Tokyo (JP)**
- **KATO, Yuki**
  **Tokyo (JP)**

(74) Representative: **WESER & Kollegen Patentanwälte PartmbB**
**Radeckestraße 43**
**81245 München (DE)**

(54) **TEST PARTICLE, REAGENT, KIT, METHOD FOR DETECTING, AND METHOD FOR PRODUCING PARTICLE**

(57) A particle for latex agglutination method that can be used as a test particle, having a reactive functional group for chemically bonding to a ligand, with a small nonspecific adsorption, excellent in dispersion stability after being stored standing still, i.e., excellent in static storage stability is provided. The test particle has a ligand, and a particle chemically bonded to the ligand, wherein the particle has a polymer including a structural unit A represented by formula (1) and a structural unit B represented by formula (2) (refer to specification) and has a specific gravity in the range of 1.00 or more and 1.10 or less, and has a composition ratio of element C to element O quantified by XPS measurement in the range of 2.1 or more and 3.3 or less.

FIG. 1

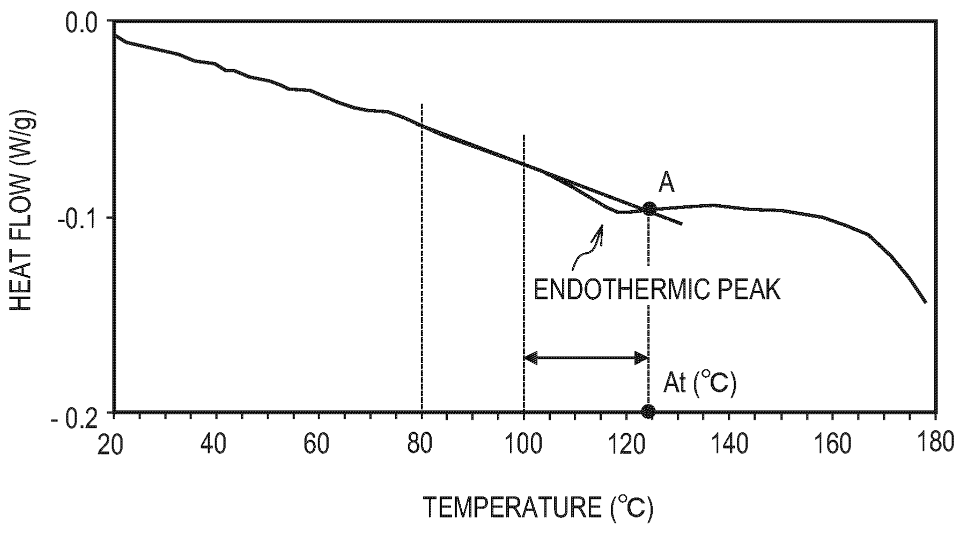


EP 4 564 003 A2

## Description

BACKGROUND OF THE INVENTION

Field of the Invention

The present invention relates to a test particle, a reagent, a kit, a method for detecting, and a method for producing a particle.

Description of the Related Art

Examples of simple and rapid immunological testing methods include a latex agglutination method. In the method, a dispersion of test particles including a latex particle bonded to a ligand having affinity with a target substance is mixed with a specimen possibly containing a target substance. On this occasion, in a case where the target substance is contained in the specimen, the test particles cause an agglutination reaction, which is optically detected as variation in the scattered light intensity, transmitted light intensity, absorbance, etc., so that disease diagnosis can be achieved.

Many of the immunological testing items in clinical test have important diagnosis points in a region where a target substance is present at a low concentration. Accordingly, it is required to be able to determine a presence or an absence of a target substance even at a low concentration. As known means for improving test sensitivity, a latex particle having a large particle size is used. Thereby, the variation in the absorbance, etc. to be detected is increased, so that measurement sensitivity in a low concentration region can be improved.

The particle used for the latex agglutination method and the test particle having a ligand for a target substance on the particle surface are required to have weak feeble adsorption to substances other than the target substance, i.e., to have small non-specific adsorption. As the particle having feeble non-specific adsorption, a particle having a surface disposed with polyglycidyl methacrylate is known. The polyglycidyl methacrylate disposed on the particle surface is presumed to have feeble non-specific adsorption, because a part of glycidyl groups is ring-opened to form glycol. In a case disclosed in Japanese Patent Application Laid-Open No. 2000-351814, a copolymerized particle of styrene and glycidyl methacrylate, of which surface disposed with polyglycidyl methacrylate is chemically bonded to a ligand through a polyethylene glycol chain, is applied to bio-separation.

The glycidyl group of glycidyl methacrylate may be also used as a reactive functional group to chemically bond a ligand to the particle surface. Use of a chemical bond to fix a ligand to the particle surface allows the concerns such as a ligand desorption from the particle surface in a case where a reagent is stored for a long term. Japanese Patent Application Laid-Open No. 2006-71297 discloses a case where a glycidyl group is carboxylated, so that a carboxy group is imparted to the particle surface to be chemically bonded to a ligand.

SUMMARY OF THE INVENTION

After study to improve detection sensitivity at a low concentration region, the present inventors have found that with further increase in a particle size of a test particle made of copolymerized styrene and glycidyl (meth)acrylate as described in Japanese Patent Application Laid-Open No. 2000-351814, particle sedimentation rate is accelerated during static storage. It is presumed that the acceleration is mainly caused due to a combination of the blending amount of glycidyl (meth)acrylate having a specific gravity of more than 1 and the particle size.

Since a dispersion of test particles may be stored standing still for several weeks depending on a test agency, a plan to maintain a dispersion of test particles in a dispersion is technically important. Occurrence of particle sedimentation during storage causes decline in dispensing accuracy during test, resulting in difficulty of precise measurement.

Specific gravity of a particle is important to maintain a dispersion of test particles. In general, a dispersion medium of a reagent for in vitro diagnostic is water. Accordingly, a difference between specific gravity of water, i.e. 1.0, and the particle specific gravity has great influence on the sedimentation level. In comparison between a particle having a specific gravity of 1.1 and a particle having a specific gravity of 1.2, a difference in sedimentation rate is twice. In order to maintain dispersion stability with decrease in sedimentation, a component to increase the specific gravity or viscosity of the dispersion medium may be also added. However, an addition may affect the detection sensitivity in some cases, so that technique is required to maintain balance of properties.

While use of glycidyl (meth)acrylate as particle component to reduce non-specific adsorption and to form a chemical bond of ligand to the particle surface is preferred, it is considered that reduction in a blending ratio of glycidyl (meth)acrylate in a particle is required to maintain dispersion stability with decrease in sedimentation. However, simple change in the blending ratio also results in change in amount ratio on a copolymerized particle surface, which may result in limitation on a detection sensibility due to decrease in an amount of bondable ligand or significant worsening of non-specific adsorption due to decrease of hydroxy groups on the surface.

The present invention has been made in the light of these background arts and problems. An object of the present invention is to provide a test particle for latex agglutination method, having a reactive functional group for chemically bonding to a ligand, with a small non-specific adsorption, excellent in dispersion stability after being stored standing still, i.e., excellent in static storage stability. Another object of the present invention is to provide a method for producing the particle. A further object of the present invention is to provide a reagent, a kit and a detection method.

In order to solve the above problem, the present invention provides a test particle having:

a ligand, and
a particle chemically bonded to the ligand,
wherein the particle has a polymer including a structural unit A represented by the following formula (1) and a structural unit B represented by the following formula (2),
the particle has a specific gravity in a range of 1.00 or more and 1.10 or less, and
the particle has a composition ratio of element C to element O quantified by XPS measurement in a range of 2.1 or more and 3.3 or less:

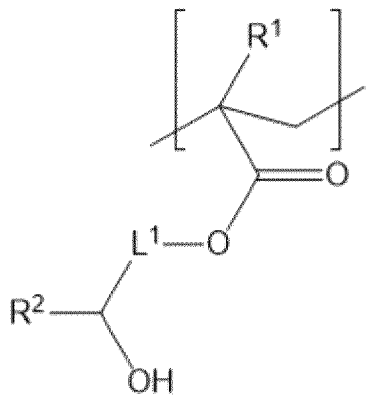

(1)

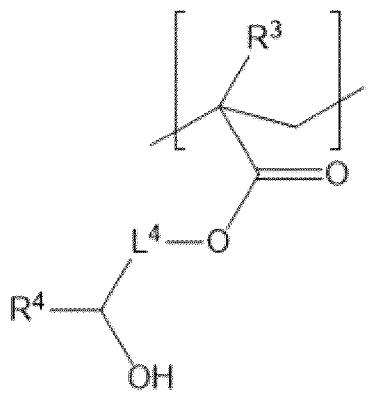

(2)

wherein, in the formula (1), $R^1$ represents a methyl group or a hydrogen atom, $L^1$ represents an alkylene group having 1 or more and 4 or less carbon atoms, and $R^2$ is a group including a sulfide group or a secondary amine and a hydroxy group; and wherein, in the formula (2), $R^3$ represents a methyl group or a hydrogen atom, $L^4$ represents an alkylene group having 1 or more and 4 or less carbon atoms, and $R^4$ is a group including a sulfide group or a secondary amine and a carboxy group.

The present invention also provides a method for producing a particle including the following steps:

step 1: mixing a compound represented by the following formula (7), a compound represented by the following formula (8), water and a radical polymerization initiator to initiate polymerization;
step 2: further adding a compound represented by the formula (7) to a reaction system after the step 1;
wherein a ratio of a mass of the compound represented by the formula (7) added in the step 2 relative to a sum of a mass of the compound represented by the formula (7) and a mass of the compound represented by the formula (8) mixed in the step 1 is 0.16 or more and 0.30 or less:

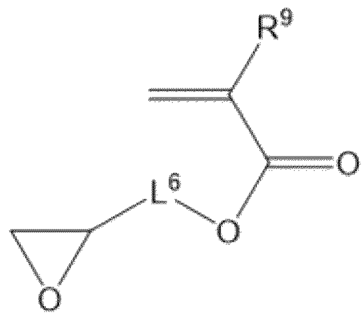

(7)

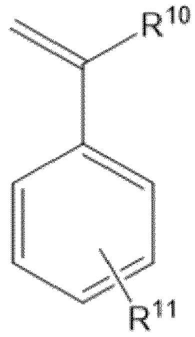

(8)

wherein, in the formula (7), $R^9$ represents a methyl group or a hydrogen atom, $L^6$ represents an alkylene group having 1 or more and 4 or less carbon atoms; and wherein, in the formula (8), $R^{10}$ represents a methyl group or a hydrogen atom, and $R^{11}$ represents a straight-chain or branched alkyl group having 1 or more and 9 or less carbon atoms or a hydrogen atom.

The present invention also provides a test particle including a particle described above chemically bonded to a ligand.

The present invention also provides a reagent for detecting a target substance in a specimen by in vitro diagnosis, containing the test particle described above.

The present invention also provides a kit for detecting a target substance in a specimen by in vitro diagnosis, including at least the reagent described above.

The present invention also provides a method for detecting a target substance in a specimen by agglutination reaction, including mixing the test particle described above with the specimen possibly containing the target substance.

Further features of the present invention will become apparent from the following description of exemplary embodiments with reference to the attached drawing.

## BRIEF DESCRIPTION OF THE DRAWINGS

FIG. 1 illustrates a DSC curve in a second temperature rise in differential scanning calorimetry (DSC) of a particle of the present invention.

## DESCRIPTION OF THE EMBODIMENTS

Preferred embodiments of the present invention will now be described in detail in accordance with the accompanying drawing.

Embodiments of the present invention are described in more details as follows, though the technical scope of the present invention is not limited to the embodiments.

[First embodiment]

A first embodiment relates to a test particle.

The test particle of the present invention has

a ligand, and
a particle chemically bonded to the ligand,

wherein the particle has a polymer including a structural unit A represented by the following formula (1) and a structural unit B represented by the following formula (2) and has a specific gravity in a range of 1.00 or more and 1.10 or less and has a composition ratio of element C to element O quantified by XPS measurement in a range of 2.1 or more and 3.3 or less:

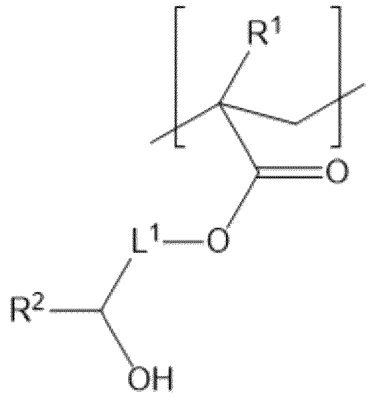

(1)

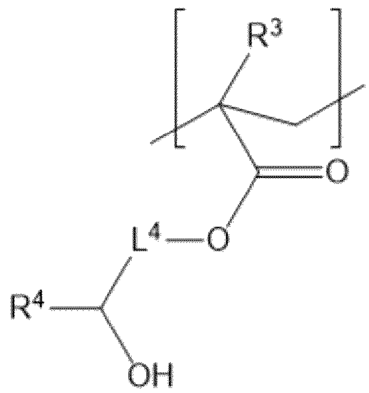

(2)

wherein, in the formula (1), $R^1$ represents a methyl group or a hydrogen atom, $L^1$ represents an alkylene group having 1 or more and 4 or less carbon atoms, and $R^2$ is a group including a sulfide group or a secondary amine and a hydroxy group; and wherein, in the formula (2), $R^3$ represents a methyl group or a hydrogen atom, $L^4$ represents an alkylene group having 1 or more and 4 or less carbon atoms, and $R^4$ is a group including a sulfide group or a secondary amine and a carboxy group.

$L^1$ in the formula (1) and $L^4$ in the formula (2) are preferably a straight-chain or branched alkylene group such as a methylene group, ethylene group, n-propylene group, isopropylene group, n-butylene group and isobutylene group, and more preferably a methylene group from a viewpoint of balancing between hydrophilicity and hydrophobicity. For that reason, it is more preferable to use glycidyl (meth)acrylate as epoxy group-containing monomer for forming a mother particle in a preliminary stage of production of the particle of the present invention.

In a case where a glycidyl group-containing monomer such as glycidyl (meth)acrylate is used to form a mother particle, a specific compound is allowed to react with the glycidyl group that is present in a mother particle surface layer. Thereby, $R^2$ in the formula (1) and $R^4$ in the formula (2) can be added to a particle surface layer as a side chain, so that a particle having a polymer including structural units A and B can be obtained.

In particular, since the side chain of the structural unit A has 3 or more hydroxy groups in total including a hydroxyl group at a terminal, and 2 or more hydroxy groups included in $R^2$, an effect of reducing non-specific adsorption of a particle is enhanced.

In the test particle of the present invention, it is preferable that the structural unit A contain a structure represented by the following formula (5):

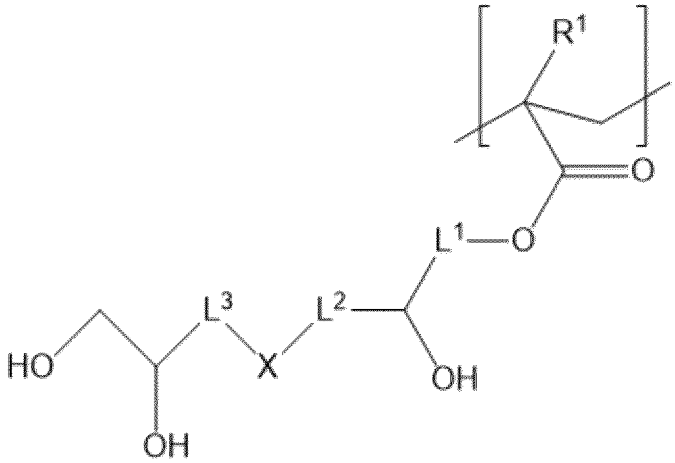

(5)

wherein $R^1$ represents a methyl group or a hydrogen atom, $L^1$ represents an alkylene group having 1 or more and 4 or less carbon atoms, $L^2$ and $L^3$ each independently represent any one of a single bond and an alkylene group having 1 or more and 3 or less carbon atoms, and X represents S or NH.

$L^1$ in the formula (5) is preferably a straight-chain or branched alkylene group such as a methylene group, ethylene group, n-propylene group, isopropylene group, n-butylene group and isobutylene group. $L^2$ and $L^3$ in the formula (5) are preferably a single bond or a straight-chain or branched alkylene group such as a methylene group, ethylene group, n-propylene group and isopropylene group. $L^1$, $L^2$ and $L^3$ in the formula (5) are more preferably a methylene group, respectively. In the case where each is a methylene group, the balance between hydrophilicity and hydrophobicity is good and further a side chain of the structural unit A is not longer than a side chain of the structural unit B, so that possibility of hindering reactivity of the carboxy group of the structural unit B can be further reduced.

X in the formula (5) may be either a sulfur atom or a nitrogen atom. Sulfur atom is more preferred from a viewpoint of improvement in sensitivity, and nitrogen atom is more preferred from a viewpoint of suppression of non-specific adsorption. Since a sulfide bond has a structure that exhibits a weak hydrophobic tendency, it is expected that water-binding force of a highly hydrophilic side chain is moderately weakened to suppress osmotic flocculation that may be caused by mixing with a specimen at high concentrations, so that the sulfide bond can contribute to improving sensitivity. On the other hand, an amino group has a structure that exhibits a hydrophilic tendency and can contribute to reduction in non-specific adsorption.

In the case where a glycidyl group derived from glycidyl (meth)acrylate is reacted with 3-mercapto-1,2-propanediol, in the formula (5), a structural unit A with $L^1$, $L^2$ and $L^3$ each representing a methylene group and X representing a sulfur atom, which is still more preferable from a viewpoint of improving the sensitivity as described above.

Also, in a case where a glycidyl group derived from glycidyl (meth)acrylate is reacted with 3-amino-1,2-propanediol, in the formula (5), a structural unit A with $L^1$, $L^2$ and $L^3$ each representing a methylene group and X representing a sulfur atom is formed, which is still more preferable from a viewpoint of reducing non-specific adsorption as described above.

The side chain of the structural unit B has a carboxy group for chemically bonding to a ligand. In particular, with having two or more carboxy groups, a reaction efficiency with the ligand is improved.

In the test particle of the present invention, it is preferable that the structural unit B contain a structure represented by the following formula (6):

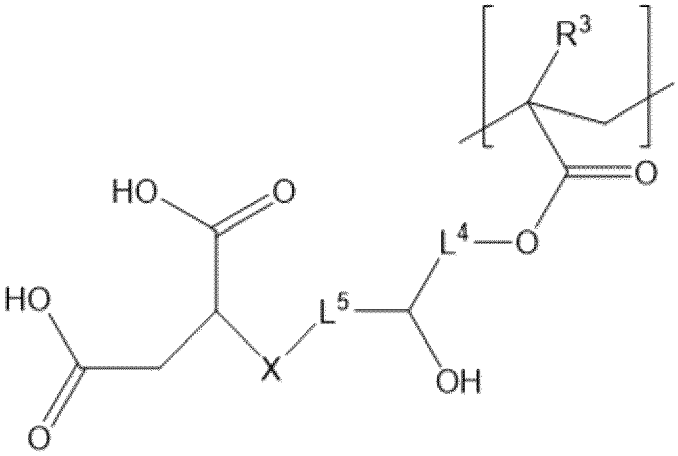

(6)

wherein $R^3$ represents a methyl group or a hydrogen atom, $L^4$ represents an alkylene group having 1 or more and 4 or less carbon atoms, $L^5$ represents any one of a single bond and an alkylene group having 1 or more and 3 or less carbon atoms, and X represents S or NH.

In the case where carboxy groups are present very close to each other as in the formula (6), interaction therebetween suppresses dissociation from each other, so that dispersibility of particles is moderately destabilized. Accordingly, use of the particle in an immunity latex agglutination measurement method is advantageous to detect a target substance with high sensitivity.

$L^4$ in the formula (6) is preferably a straight-chain or branched alkylene group such as a methylene group, ethylene group, n-propylene group, isopropylene group, n-butylene group and isobutylene group. $L^5$ in the formula (6) is preferably a single bond or a straight-chain or branched alkylene group such as a methylene group, ethylene group, n-propylene group and isopropylene group. $L^4$ and $L^5$ in the formula (6) are more preferably a methylene group. With a group having too many carbon atoms, this has a risk that a non-specific adsorption to a particle is facilitated due to increase in hydrophobicity.

X in the formula (6) may be either a sulfur atom or a nitrogen atom, and a sulfur atom is more preferred. Since a sulfide bond has a structure that exhibits a weak hydrophobic tendency, it is expected that a water binding force of a side chain is moderately weakened to suppress osmotic flocculation that may be caused by mixing with a specimen at high concentrations.

In a case where a glycidyl group derived from glycidyl (meth)acrylate is reacted with mercaptosuccinic acid, a structural unit B is formed with $L^4$ and $L^5$ each in the formula (6) being a methylene group and X being a sulfur atom, which is still more preferred as described above.

It is preferable that [Structural unit A]/[Structural unit B] is 0.2 or more and 20 or less (molar fraction). With a molar fraction of less than 0.2, the proportion of the carboxy group contained in the structural unit B is excessive, which may damage the dispersion stability of particles and in a case of chemical bonding to a ligand, unreacted carboxy groups may interact with the ligand to degenerate the ligand. Alternatively, with a molar fraction of more than 20, due to a small proportion of the carboxy group, the reaction efficiency in chemical bonding to a ligand may decrease, or electrostatic repulsion to contribute dispersion stability of particles may decrease in some cases.

From a viewpoint of the mechanical strength of a particle, it is preferable that a particle of the present invention have a structural unit C represented by the following formula (3) or the following formula (4), and a total amount of the structural unit A and the structural unit B in a total amount of the structural unit A, the structural unit B and the structural unit C is preferably 43 mol% or less and more preferably 5 mol% or more and 43 mol% or less:

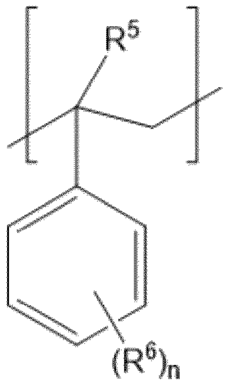

(3)

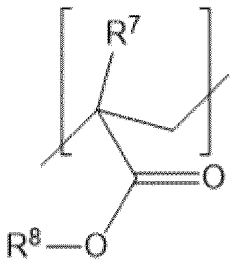

(4)

wherein, in the formula (3), $R^5$ represents a methyl group or a hydrogen atom, $R^6$ represents a straight-chain or branched alkyl group having 1 or more and 9 or less carbon atoms or a hydrogen atom, and n represents an integer of 1 or more and 5 or less; and wherein, in the formula (4), $R^7$ represents a methyl group or a hydrogen atom, and $R^8$ represents a straight-chain or branched alkyl group having 1 or more and 12 or less carbon atoms.

Due to containing the structural unit C, the particle of the present invention is physically strengthened, so that damages to the particle such as cracks and chips can be suppressed even when operations such as centrifugation are repeated in a refining process of the particle. Further, containing the structural unit C is preferred from a viewpoint of decrease in a specific gravity of the particle.

Examples of the compound from which the components represented by the formula (3) are derived include styrenes such as styrene, α-methylstyrene, o-methylstyrene, m-methylstyrene, p-methylstyrene, 2,4-dimethylstyrene, p-n-butylstyrene, p-tert-butylstyrene, p-n-hexylstyrene, p-n-octylstyrene and p-n-nonylstyrene.

Examples of the compound from which the components represented by the formula (4) are derived include (meth)acrylates such as methyl acrylate, ethyl acrylate, n-propyl acrylate, iso-propyl acrylate, n-butyl acrylate, iso-butyl acrylate, tert-butyl acrylate, n-amyl acrylate, n-hexyl acrylate, 2-ethylhexyl acrylate, n-octyl acrylate, n-nonyl acrylate, cyclohexyl acrylate, methyl methacrylate, ethyl methacrylate, n-propyl methacrylate, iso-propyl methacrylate, n-butyl methacrylate, iso-butyl methacrylate, tert-butyl methacrylate, n-amyl methacrylate, n-hexyl methacrylate, 2-ethylhexyl methacrylate, n-octyl methacrylate and n-nonyl methacrylate.

In any case, the compound is not limited thereto as long as a purpose of the invention can be achieved. Alternatively, two or more components may be used in a combination.

The compound from which the structural unit C is derived is preferably styrenes represented by the formula (3), and more preferably styrene. Further, the structural unit C of the particle of the present invention is preferably at least one selected from the group consisting of styrenes, more preferably styrene. The monomers of styrenes have a small specific gravity and excellent refractive index, being preferable from a viewpoint of reducing the specific gravity of the particle and from a viewpoint of improving the refractive index of the particle to improve sensitivity.

In the particle of the present invention, an amount of the structural unit C in the total amount of the structural unit A, the structural unit B and the structural unit C is preferably 57 mol% or more and 95 mol% or less, and still more preferably 68 mol% or more and 93 mol% or less. (In other words, as described above, a total amount of the structural unit A and the structural unit B in a total amount of the structural unit A, the structural unit B and the structural unit C is preferably 5 mol% or more and 43 mol% or less.)

<Specific gravity of particle of the present invention>

The specific gravity of the particle of the present invention is in a range of 1.00 or more and 1.10 or less. With a specific gravity of 1.10 or less, excellent dispersion stability can be maintained even in a case where the dispersion of the test particle as a test reagent is stored standing still. Since sedimentation of the particle is reduced, the usable period of the

test reagent can be prolonged. Specifically, dispensing accuracy can be maintained even after storage for a specific period, so that decrease in the detection sensitivity can be prevented.

The dominant factors that determine the specific gravity of a particle include constituent components of the particle. Further, in a case of a particle having cross-linking properties, the degree of cross-linking above a certain level also influences the specific gravity.

The constituent components of the particle of the present invention are mainly components derived from a reactive monomer for use in forming the mother particle described above. Specifically, since the main components are a glycidyl group-containing monomer from which the structural units A and B are derived (in the present invention, more preferably glycidyl (meth)acrylate) and a monomer from which the structural unit C is derived (in the present invention, more preferably styrene), the specific gravity of the particle of the present invention is approximately determined by the content ratio of these monomers to form a mother particle.

In other words, the side chain components and the ligand that are added to the surface after forming a mother particle are present only in a vicinity of the particle surface, with very small proportions in the constituent components of the particle, so that the contribution to the specific gravity of the particle of the present invention is very small.

The specific gravity of a particle increases in proportion to the content of a glycidyl group-containing monomer having a specific gravity of more than 1 (in the present invention, more preferably glycidyl (meth)acrylate) among monomers for forming a mother particle. Accordingly, in order to maintain the dispersion stability with decrease in sedimentation, it is preferable that a content of epoxy group-containing monomers is reduced in formation of a mother particle. Specifically, the total amount of the structural unit A and the structural unit B in the total amount of the structural unit A, the structural unit B and the structural unit C is preferably 43 mol% or less, and still more preferably 32 mol% or less.

On the other hand, structures of the structural units A and B are essential for excellent non-specific adsorption and chemical bonding of the ligand to the particle surface, and with a too small amount may result in worsened non-specific adsorption and bonding to insufficient amount of ligands. Accordingly, a total amount of the structural unit A and the structural unit B in a total amount of the structural unit A, the structural unit B and the structural unit C is preferably 20 mol% or more.

<Composition ratio quantified by XPS measurement of particle of the present invention>

As described above, while glycidyl (meth)acrylate is preferably used as a component of the particle to reduce the non-specific adsorption and to chemically bond a ligand to the particle surface, it is presumed that the content of glycidyl (meth)acrylate in forming of the particle needs to be reduced for maintaining the dispersion stability with sedimentation decreased. However, simple reduction in a blending amount reduces not only the amount over the whole region of the particle but also an amount in a "surface layer" of the particle. In that case, due to a decrease in amounts of carboxy groups and hydroxy groups imparted in a post-process, an amount of bondable ligands may decrease, or non-specific adhesion may be significantly worsened.

It is preferable that the particle of the present invention is a particle with an amount ratio in the "surface layer" of the particle appropriately controlled even though an amount ratio of components in a whole region of the particle, i.e. a preparation ratio, is changed. (A method for producing a particle with proper control is described as follows.)

The particle of the present invention is a particle of which composition ratio of element C to element O quantified by XPS measurement is controlled in a range of 2.1 or more and 3.3 or less. In XPS measurement, photoelectrons generated in a region from an outermost surface to about 10 nm are detected, so that the components in the "surface layer" of a particle can be analyzed. In a case where a composition ratio is less than 2.1, elements O in the "surface layer" of the particle are excessive, so that sufficient amounts of hydroxy groups and carboxy groups are added. On the other hand, due to very high hydrophilicity, agglutination of particles hardly occurs, so that detection sensitivity based on a principle of the latex agglutination method may decrease. In contrast, in the case where the composition ratio is more than 3.3, elements O in the "surface layer" of the particle are too few, so that hydrophilicity is insufficient, resulting in not only disadvantage in dispersion stability of particles during long-term storage but also worsened non-specific adsorption. As described above, through optimization of the amount of components in the "surface layer" of a particle, a particle excellent in both the detection sensitivity and the non-specific adhesion can be provided.

<DSC curve of particle of the present invention>

On the DSC curve obtained in a second temperature rise in differential scanning calorimetry (DSC) measurement of the particle of the present invention, a straight line passing through a point on the DSC curve at a temperature of 80°C and a point on the DSC curve at a temperature of 100°C is drawn. An intersection point between the straight line and the DSC curve in the temperature region of 105°C or more and 140°C or less is defined as intersection point A. It is preferable that the DSC curve have an endothermic peak in a temperature region between a temperature At of the intersection point A and 100°C.

An endothermic peak is derived from properties of styrenes (structural unit C of the present invention). The endothermic peak thus observed indicates a region where polystyrenes congregate at high concentrations in a particle, and the structural units A and B and polystyrene (structural unit C) are constitutively separated in a particle. In the particle of the present invention, since the structural units A and B are mainly disposed in a surface layer to control the "surface layer", the polystyrene (structural unit C) on the other hand is presumed to be confined inside the particle. Since the presence of polystyrene as hydrophobic material in the surface layer of a particle can be a main factor to worsen non-specific adsorption, through such firm confinement of polystyrene inside a particle, a particle excellent in non-specific adsorption can be provided.

In a case where the structural unit C of the present invention contains a polymethyl (meth)acrylate-based structure represented by the formula (4), a straight line passing through a point at a temperature of 80°C and a point at a temperature of 100°C on the DSC curve obtained in a second temperature rise in DSC measurement is drawn. It is preferable that the straight line and the DSC curve have an intersection point A in a region of 95°C or more and 140°C or less, with an endothermic peak in a temperature region between a temperature At of the intersection point A and 90°C.

<Zeta potential of particle of the present invention>

It is preferable that the particle of the present invention have a zeta potential of -50 mV or more and -10 mV or less. A zeta potential of less than -50 mV indicates that the surface layer of a particle has many carboxy groups. In a case where a particle is bonded to a ligand to make a test particle, reactivity of the ligand may be reduced due to electrostatic interaction between the ligand and the particle surface, or electric charge deviation on the surface of the test particle due to zeta potential difference between the ligand and the particle surface may cause heteroagglutination. In order to reduce possibility, zeta potential is preferably -50 mV or more. A zeta potential of more than -10 mV indicates that the surface layer of a particle has a few carboxy groups. In order to bond a sufficient amount of ligands to a particle to achieve excellent detection sensitivity, the zeta potential is preferably -10 mV or less.

<Particle size of particle of the present invention>

The particle of the present invention has a volume average particle size in water dispersion of preferably 50 nm or more and 500 nm or less, more preferably 280 nm or more and 400 nm or less, and still more preferably 280 nm or more and 350 nm or less.

With a particle size of 280 nm or more, variation in absorbance, etc. detected by latex agglutination method increases, so that detection sensitivity is improved particularly in a case where a target substance is present at a low concentration. Conventionally, with use of a particle having a size of 280 nm or more and containing a component derived from glycidyl (meth)acrylate as a test reagent, problems in practical use tend to occur in a long-term storage of a test reagent due to particle sedimentation resulting from size of the particle and the specific gravity of glycidyl (meth)acrylate. Use of the particle of the present invention, of which specific gravity is controlled, can improve the dispersion stability of the test reagent and particle sedimentation in a long-term storage, even in the case where the particle has a size of 280 nm or more. From a same viewpoint, the particle size is more preferably 400 nm or less, and still more preferably 350 nm or less.

[Second embodiment]

The second embodiment relates to a method for producing a particle.

As described above, while use of glycidyl (meth)acrylate as component of the particle is preferred, it is presumed that decrease in a content of glycidyl (meth)acrylate is required in particle formation. However, simple decrease in the blending amount results in not only decrease in an amount in the whole region of the particle, but also decrease in the amount in the "surface layer" of the particle.

It is preferable that the particle of the present invention is a particle with an amount ratio in the "surface layer" appropriately controlled even when the amount ratio of the components in the whole region of the particle, i.e. a preparation ratio, is changed. In order to achieve the requirement, the method for producing the particle of the present invention includes the following steps.

step 1: mixing a compound represented by the following formula (7), a compound represented by the following formula (8), water and a radical polymerization initiator to initiate polymerization;
step 2: further adding a compound represented by the formula (7) to a reaction system after the step 1;
wherein a ratio of a mass of the compound represented by the formula (7) added in the step 2 relative to a sum of a mass of the compound represented by the formula (7) and a mass of the compound represented by the formula (8) mixed in the step 1 is 0.16 or more and 0.30 or less:

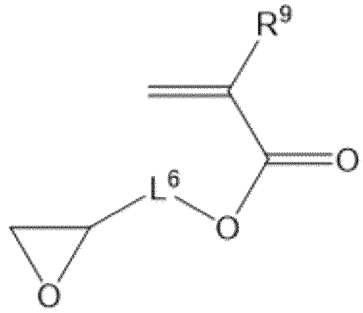

(7)

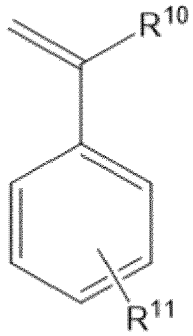

(8)

wherein, in the formula (7), $R^9$ represents a methyl group or a hydrogen atom, and $L^6$ represents an alkylene group having 1 or more and 4 or less carbon atoms; and wherein, in the formula (8), $R^{10}$ represents a methyl group or a hydrogen atom, and $R^{11}$ represents a straight-chain or branched alkyl group having 1 or more and 9 or less carbon atoms or a hydrogen atom.

$L^6$ in the formula (7) is preferably a straight-chain or branched alkylene group such as a methylene group, ethylene group, n-propylene group, isopropylene group, n-butylene group and isobutylene group. $R^{11}$ in the formula (8) is preferably a methyl group, ethyl group, propyl group, isopropyl group, n-butyl group, isobutyl group, sec-butyl group, tert-butyl group, n-pentyl group, tert-pentyl group, neopentyl group, n-hexyl group, n-heptyl group, n-octyl group and n-nonyl group.

The compound represented by the formula (7) added in step 2, i.e. a glycidyl group-containing monomer, is immediately dissolved after addition to water of a reaction system to be used in a polymerization reaction and attached to the surface layer of the particle.

With an amount of the compound represented by the formula (7), i.e. glycidyl group-containing monomer, added in the step 2 controlled to 16 mass% or more, a sufficient amount of glycidyl groups can be present in the "surface layer" of a mother particle even in a case where a total amount of glycidyl group-containing monomers used for forming a mother particle is reduced. Thereby, sufficient amounts of hydroxy groups and carboxy groups can be added to the surface layer of a particle in a post-process. A composition ratio of element C to element O becomes 3.3 or less, so that a particle having high hydrophilicity and excellent non-specific adsorption can be provided. Further, with an amount of glycidyl group-containing monomer added in the step 2 controlled to 30 mass% or less, the glycidyl groups in the surface layer of a mother particle do not increase too much, so that excessive amounts of hydroxy groups and carboxy groups can be prevented from being added to the surface layer of a particle. The composition ratio of element C to element O quantified by XPS measurement of a particle becomes 2.1 or more to provide appropriate hydrophobicity, so that agglutination properties required for the latex agglutination method can be secured and a particle excellent in detection sensitivity can be provided.

In a method for producing a particle of the present invention, it is preferable that addition of a compound represented by the formula (7), i.e. glycidyl group-containing monomer, is performed in several batches in the step 2. In order to more accurately control an amount of glycidyl groups in the surface layer of a mother particle, an amount of monomers remaining in a reaction system in adding in the step 2 needs to be considered. In a case where the addition of monomers is performed in several batches in the step 2, the monomers remaining in the reaction system (in particular, styrenes having a small solubility in water) can be efficiently dissolved in water to facilitate a polymerization reaction in first addition. Thereby, in adding in the step 2, an amount of monomers remaining in the reaction system can be reduced, so that dissolving of the remaining monomers at the last time of addition is reduced. Accordingly, a ratio of the glycidyl groups finally present in the "surface layer" of a particle can be efficiently increased. "Addition of monomers is performed in several batches" means that a first addition and a final addition are separated for 1 minute or more. As a specific method, a lump amount may be added several times at intervals, or continuous dripping may be performed without intervals.

The method for producing a particle of the present invention preferably includes the following step 3 after the step 2.

Step 3: mixing a water dispersion of the resulting granular copolymer, 3-mercapto-1,2-propanediol and mercaptosuccinic acid to prepare a mixture, thereby reacting an epoxy group derived from the compound represented by the formula (7) with a thiol group derived from 3-mercapto-1,2-propanediol and mercaptosuccinic acid.

Through a reaction of an epoxy group of the resulting granular copolymer (mother particle) with 3-mercapto-1,2-propanediol and mercaptosuccinic acid, the side chains represented by the structural unit A represented by the formula (5) and the structural unit B represented by the formula (6) can be imparted to the surface layer of a particle. The reason why 3-mercapto-1,2-propanediol and mercaptosuccinic acid are more preferred is as described above.

In the method for producing a particle of the present invention, other typical production methods are described as follows. However, a method for producing a particle of the present invention is not limited thereto within a range where an object of the present invention can be achieved.

The radical polymerization initiator in the step 1 is preferably a water-soluble polymerization initiator.

Although a water-soluble polymerization initiator is not particularly limited, a water-soluble azo compound and a water-soluble peroxide are preferably used.

The water-soluble azo compound is preferably any of 4,4'-azobis(4-cyanovaleric acid), 2,2'-azobis[2-methyl-N-(2-hydroxyethyl)propionamide], 2.2'-azobis[N-(2-carboxyethyl)-2-methylpropionamidine]tetrahydrate, 2,2'-azobis(2-methylpropionamidine)dihydrochloride, 2,2'-azobis[2-(2-imidazoline-2-yl)propane], and 2,2'-azobis[2-(2-imidazoline-2-yl)propane]disulfate dihydrate.

The water-soluble peroxide is preferably any of potassium persulfate, ammonium persulfate, sodium persulfate, tertiary butyl hydroperoxide, cumyl hydroperoxide, paramenthane hydroperoxide, and di-isopropyl benzene hydroperoxide.

In the step 1, a radical-polymerizable monomer having cross-linkability may be further used. Examples of the radical-polymerizable monomer having cross-linkability include diethylene glycol diacrylate, triethylene glycol diacrylate, tetraethylene glycol diacrylate, polyethylene glycol diacrylate, 1,6-hexane diol diacrylate, neopentyl glycol diacrylate, tripropylene glycol diacrylate, polypropylene glycol diacrylate, 2,2'-bis(4-(acryloxy diethoxy)phenyl)propane, trimethylol propane triacrylate, tetramethylol methane tetraacrylate, ethylene glycol dimethacrylate, diethylene glycol dimethacrylate, triethylene glycol dimethacrylate, tetraethylene glycol dimethacrylate, polyethylene glycol dimethacrylate, 1,3-butylene glycol dimethacrylate, 1,6-hexane diol dimethacrylate, neopentyl glycol dimethacrylate, polypropylene glycol dimethacrylate, 2,2'-bis(4-(methacryloxy diethoxy)phenyl)propane, 2,2'-bis(4-(methacryloxy polyethoxy)phenyl)propane, trimethylol propane trimethacrylate, tetramethylol methane tetramethacrylate, divinyl benzene, divinyl naphthaline, and divinyl ether, though not limited thereto within a range where the object of the present invention can be achieved. Further, two or more radical-polymerizable monomers having cross-linkability may be used in combination.

An amount of the radical-polymerizable monomer having cross-linkability is preferably from 0.1 mass% to 5 mass% of the whole monomers for use in polymerization. With an amount of more than 5 mass%, the particle density increases, so that a specific density of the particle may increase.

In a case where a granular copolymer (mother particle) is formed by radical polymerization, emulsion polymerization, soap-free emulsion polymerization, and suspension polymerization are preferably used, and emulsion polymerization or soap-free emulsion polymerization is more preferably used. Still more preferably, soap-free emulsion polymerization is used. In general, a granular polymer obtained by emulsion polymerization or soap-free emulsion polymerization has a sharper particle size distribution, in comparison with those obtained by suspension polymerization. In a case where a particle bonded to a ligand is used as a test particle, a concern is that an anionic surfactant or cationic surfactant usually used in emulsion polymerization present as residue may denature the ligand. For this reason, in a case where a granular polymer (mother particle) is formed by emulsion polymerization, use of a nonionic surfactant is preferred.

The step 3 includes adjusting the pH to an alkaline region with an organic base having no primary amine. In the step 3, a reaction of the epoxy group that the granular copolymer (mother particle) has is caused from the surface of the granular copolymer (mother particle) in the depth direction using 3-mercapto-1,2-propanediol and the thiol group derived from mercaptosuccinic acid.

In order to cause a sufficient reaction in the depth direction, it is preferable that triethylamine that has permeability into the granular copolymer (mother particle) is selected as organic base.

It is preferable that the method for producing a particle of the present invention include the following step 3' after the step 2.

Step 3': mixing a water dispersion of the resulting granular copolymer, 3-amino-1,2-propanediol and mercaptosuccinic acid to prepare a mixture, thereby reacting an epoxy group derived from the compound represented by the formula (7) with an amino group derived from 3-amino-1,2-propanediol and a thiol group derived from mercaptosuccinic acid.

[Application example]

Examples of the application examples of the present invention include a test particle, a reagent, a kit and a detection method. Each item is described as follows.

<Test particle>

The test particle of the present invention preferably includes the particle described above chemically bonded to a ligand, and more preferably includes the particle described above bonded to a ligand having affinity with a target substance. It is preferable that the test particle of the present invention is used in detection of a target substance in a specimen by the agglutination method.

The ligand refers to a compound that bonds specifically to a receptor of a specific target substance. A region where the ligand bonds to a target substance is specific and has selectively or specifically high affinity. Examples of the ligand include antigen and antibody, enzyme protein and substrate thereof, signal substance typified by hormone and neurotransmitter and receptor thereof, nucleic acid, and avidin and biotin etc., though not limited thereto in a range where the purpose of the present invention can be achieved. Specific examples of the ligand include an antigen, antibody, fragment bonding to antigen (for example, Fab, F(ab')2, F(ab'), Fv and scFv etc.), naturally derived nucleic acid, artificial nucleic acid, aptamer, peptide aptamer, oligopeptide, enzyme and coenzyme etc. The ligand of the test particle of the present invention is preferably an antibody or antigen.

In the present invention, as the chemical reaction method for bonding a carboxy group or a carboxylate derived from the structural unit B to a ligand, a conventionally known method may be applied in the range where the purpose of the present invention can be achieved. The preferred example of the chemical reaction includes for example a carbodiimide-mediated reaction and an NHS ester activation reaction. Alternatively, a carboxy group bonding to avidin may be bonded to a ligand modified with biotin. However, the chemical reaction method for bonding a carboxy group or carboxylic acid derived from the structural unit B to a ligand is not limited thereto, in the range where the purpose of the present invention can be achieved.

As described above, regarding the particle of the present invention, it can be determined that the specific gravity is kept the same between the particle before bonding to a ligand and the test particle having the chemically bonded ligand. This is because a proportion of the ligand relative to the particle is sufficiently small.

<Reagent>

It is preferable that the reagent of the present invention is a reagent used for detecting a target substance in a specimen by in vitro diagnosis, containing the above-mentioned test particle and dispersion medium.

In the present invention, a case of using an antibody (antigen) as ligand and using an antigen (antibody) as target substance may be extremely preferably applied to a latex agglutination method in immunological testing widely utilized in a field such as clinical test and biochemical research, as a method for detecting a target substance in a specimen in in vitro diagnosis. In a case of using a commonly used particle as particle for the latex agglutination method, a concern is that the antigen (antibody) as target substance and foreign substances in serum or plasma are nonspecifically adsorbed to the particle surface to cause an unintentional agglutination among particles, which damages precision of the immunological testing.

It is preferable that the reagent of the present invention is used in detection of a target substance in a specimen by the agglutination method and contain a particle for the latex agglutination method. An amount of the particle for the latex agglutination method contained in the reagent of the present invention is preferably in the range of 0.001 mass% to 20 mass%, and more preferably in the range of 0.01 mass% to 10 mass%. The reagent of the present invention may contain a third substance such as solvent and blocking agent other than the particle for the latex agglutination method, in the range where the purpose of the present invention can be achieved. Examples of the solvent for use in the present invention include various water-based buffer solutions such as phosphate buffer solution, glycine buffer solution, Good's buffer solution, Tris buffer solution, HEPES buffer solution, MES buffer solution and ammonia buffer solution, though the solvent contained in the reagent of the present invention is not limited thereto.

<Kit>

The kit of the present invention is a kit for detecting a target substance in a specimen by in vitro diagnostic, preferably including at least the reagent described above. The kit of the present invention preferably further includes a reaction buffer solution containing albumin (hereinafter, referred to as reagent 2) in addition to the reagent of the present invention (hereinafter, referred to as reagent 1). Examples of the albumin include serum albumin etc., and one treated with protease may be included. An estimated amount of albumin contained in the reagent 2 is in the range of 0.001 mass% to 5

mass%, though the kit of the present invention is not limited thereto. A sensitizer for latex agglutination measurement may be contained in both the reagent 1 and the reagent 2, or any one thereof. Examples of the sensitizer for latex agglutination measurement include polyvinyl alcohol, polyvinyl pyrrolidone, polyethylene glycol and alginic acid, though not limited thereto for the kit of the present invention. Further, the kit of the present invention may include a positive control, negative control, serum diluent, etc. in addition to the reagent 1 and the reagent 2. A medium for the positive control or negative control may include a solvent in addition to a serum containing no measurable target substance and a normal saline solution. The kit of the present invention may be used in the method for detecting a target substance of the present invention, in the same manner as the kit for detecting a target substance in a specimen by normal in vitro diagnostic. Further, the kit may be also used for measuring the concentration of a target substance by a conventionally known method, being particularly suitable for detecting a target substance in a specimen by the latex agglutination method.

<Method for detecting>

A method for detecting a target substance in a specimen by in vitro diagnostic preferably includes mixing the test particle described above with a specimen possibly containing a target substance, and is more preferably a method for detecting a target substance in a specimen by agglutination reaction. Further, it is preferable that mixing of the test particle of the present invention and the specimen is performed in a range of pH 3.0 to pH 11.0. A mixing temperature is in a range of 20°C to 50°C, and a mixing time is in a range of 1 minute to 20 minutes. In the present detection method, it is preferable that a solvent is used. In the detection method of the present invention, the concentration of the test particle of the present invention is preferably 0.001 mass% to 5 mass%, and preferably 0.01 mass% to 1 mass%, in a reaction system. The detection method of the present invention is characterized by optically detecting the agglutination among particles resulting from mixing of the test particle of the present invention and a specimen. Through optical detection of the agglutination among particles, a target substance in the specimen is detected, and further, the concentration of the target substance can be also measured. In order to optically detect the agglutination reaction, the variation of scattered light intensity, transmitted light intensity, absorbance, etc. may be measured with an optical apparatus capable of detecting them.

[Method for measuring specific gravity of particle]

The method for measuring the specific gravity of a particle of the present invention is described. The particles are dispersed at 0.5 mass% in ion-exchanged water, and the sedimentation rate of the particles is measured under a specified centrifugal force. The specific gravity of the particle is calculated from the measured sedimentation value, the specific gravity and viscosity of the dispersion medium, and the volume average particle size of the particle. The ion-exchanged water for use has an electric conductivity of 10 μS/cm or less. Specifically, LUMISizer (manufactured by MS Scientific Co., Ltd.) is used to measure the sedimentation rate determined by the change in transmitted light when a centrifugal force at 4000 rpm is applied to the dispersion of the particles. Using the specific gravity and viscosity of the ion-exchanged water as dispersion medium, and the volume average particle size of the particle measured by the method described as follows, the specific gravity of the particle is calculated from the Stokes' formula shown as follows. The measurement is performed three times, and the average of the three measurement values is employed as the specific gravity.

(Stokes' formula)

$$\text{Sedimentation rate } V_s = D_\rho^2(\rho_p - \rho_f)g/18\eta$$

wherein Vs represents sedimentation rate (m/s), $D_\rho$ represents particle size (m), $\rho_p$ represents specific gravity of particle (kg/m$^3$), $\rho_f$ represents specific gravity of dispersion medium (kg/m$^3$), g represents gravitational acceleration (m/s$^2$), and $\eta$ represents viscosity of dispersion medium (Pa·s).

[Method for measuring C/O ratio of particle]

The quantitative method of the composition ratio by XPS measurement of a particle of the present invention is described. In XPS measurement of the composition ratio of a particle of the present invention, the particle in a freeze-dried state is fixed to an indium foil. The measurement apparatus and measurement conditions are as follows.

- · Measurement apparatus: X-ray photoelectron spectroscopy Quantum 2000 (trade name, manufactured by ULVAC-PHI, Inc.)
- · X-ray source: monochrome AlKα
- · X-ray setting: 100 μm ϕ (25 W (15 KV)

· Photoelectron takeoff angle: 45 degrees
· Neutralization condition: Combination use of neutralization gun and ion gun
· Analysis area: 300 μm by 200 μm
· Pass energy: 58.70 eV
· Step size: 0.125 eV
· Analysis software: MultiPak (PHI, Inc.)

In measurement, cumulative numbers $C1_s$ and $O1_s$ are 15, and $N1_s$ is 30. From resulting quantitative values of elements (composition ratio (atomic%) of element C and composition ratio (atomic%) of element O relative to a total amount of three elements), the composition ratio of element C relative to element O is determined.

[Acquisition method of DSC curve of particle]

The acquisition method of DSC curve of a particle of the present invention is described. The measurement of a sample of freeze-dried particles is performed using a differential scanning calorimeter (DSC) (trade name "Discovery DSC2500", manufactured by TA instruments). A sample sealed in an aluminum pan is measured with use of a temperature program described as follows. According to the temperature program, initially, after first temperature rising from 20°C to 180°C at a heating rate of 10°C/minute, the temperature is kept at 180°C for 10 minutes, then cooled to 10°C at a cooling rate of 10°C/minute, then kept at 10°C for 10 minutes, and subsequently second temperature rising is performed again to 180°C at a heating rate of 10°C/minute. A DSC curve during the second temperature rising is acquired.

[Method for measuring zeta potential of particle]

The method for measuring zeta potential of particle in the present invention is described. In the present invention, the zeta potential is measured in a state where the particles are dispersed at 0.001 mass% in 0.01 N potassium aqueous solution having a pH 7.8. The 0.01 N potassium aqueous solution having a pH 7.8 for use is obtained by appropriately mixing 0.01 N potassium chloride aqueous solution, 0.01 N potassium hydroxide aqueous solution, and 0.01 N hydrochloric acid aqueous solution, which are prepared with use of an ion-exchanged water having an electric conductivity of 10 μS/cm or less. The measurement apparatus for use is Zetasizer (Nano-ZS, manufactured by Spectris), and the measurement is performed at 25°C. The selection of analysis parameters includes latex (n≅1.59) as refractive index of particle, and purified water as dispersion medium. The measurement is performed 10 times, and the average value in the 10 times measurements is employed as the zeta potential.

[Method for measuring volume average particle size of particles in water dispersion]

The method for measuring volume average particle size of particles in water dispersion in the present invention is described. In the present invention, volume average particle size of particles in water dispersion is measured in a condition where the particles are dispersed at 0.001 mass% in an ion-exchanged water having an electric conductivity of 10 μS/cm or less. For the measurement of particle size in water, dynamic light scattering method is used. Specifically, Zetasizer (Nano-ZS, manufactured by Spectris) is used, and the measurement is performed at 25°C. The selection of analysis parameters includes latex (n≅1.59) as refractive index of particle, and purified water as dispersion medium. The measurement is performed 10 times, and an average value in the 10 times measurements is employed as the particle size in water.

[Method for measuring antibody sensitization rate of test particle]

The method for measuring antibody sensitization rate of test particle prepared from the particle of the present invention is described. The antibody sensitization rate (%) of test particle is determined by protein quantification. The antibody sensitization rate (%) means the proportion of an amount of antibody bonded to the particle relative to an amount of antibody used for the reaction (preparation amount of antibody).

First, 7 mL of liquid A and 140 μL of liquid B of Protein assay BCA kit (manufactured by Wako Pure Chemical) are mixed to prepare a liquid AB. Subsequently, to 25 μL (amount of particles: 25 μg) of a dispersion of the test particles (0.1% solution), 200 μL of liquid AB is added, and the mixture is incubated at 60°C for 30 minutes. The solution is subjected to centrifugation at 4°C and 15000 rpm (20400 g), for 5 minutes, and 200 μL of supernatant is put in a 96-hole microwell with a pipette. The specimen and standard samples (several samples of antibody in the range of 0 to 200 μg/mL in 10 mM HEPES) are subjected to measurement of absorbance at 562 nm with a microplate reader, so that an amount of antibody is calculated from the standard curve. The sensitization amount of antibody to the particle (the amount of antibody bonded per mass of particle (μg/mg)) is determined by dividing the calculated amount of antibody by a mass of particle (in this case,

0.025 mg). Finally, the sensitization rate is calculated. In a case where the preparation amount of antibody is 25 μg per 1 mg of particles and a sensitization amount of antibody is 12.5 μg/mg, the sensitization rate is 50%.

[Examples]

The present invention is described in more detail with reference to Examples as follows, though the present invention is not limited to these Examples.

[Example 1-1]

(Synthesis of granular copolymer (mother particle) 1)

Into a 2L four-neck separable flask, 27.8 g of styrene (St, manufactured by Kishida Chemical Co., Ltd.), 17.3 g of glycidyl methacrylate (GMA, manufactured by Kishida Chemical Co., Ltd.), 0.70 g of divinyl benzene (DVB, manufactured by Kishida Chemical Co., Ltd.), and 1305.5 g of ion-exchanged water were weighed out to make a mixture. While stirring the mixture at 200 rpm at 70°C, nitrogen was flown at a flow rate of 200 ml/minute to purge oxygen in four-neck separable flask. Subsequently, a separately prepared solution including 0.68 g of V-50 (manufactured by FUJIFILM Wako Pure Chemical Corporation) dissolved in 18 g of ion-exchanged water was added to the mixture, so that soap-free emulsion polymerization was initiated.

After 2 hours from initiation of the polymerization, as an additional GMA step, 5.2 g of GMA was added into the four-neck separable flask, and after 30 minutes, 5.2 g of GMA was further added (5.2 g of GMA was added twice). While stirring for 22 hours at 200 rpm, the temperature was kept at 70°C, so that a water dispersion containing a granular copolymer 1 was obtained. After slowly cooling the dispersion to room temperature, a part of the dispersion was collected to evaluate the polymerization conversion rate with proton NMR and gas chromatography. As a result, the polymerization conversion rate was confirmed to be substantially 100%. In other words, a mole ratio of GMA relative to a total of St and GMA in the granular copolymer 1 is 42.2 mol%. Also, a mass ratio of an amount of GMA added relative to an amount of monomer at the start of polymerization is 0.23. A volume average particle size of the granular copolymer 1 was 240 nm. The granular copolymer 1 was stored under light-shielding conditions at 4°C.

[Example 1-2]

(Synthesis of granular copolymer (mother particle) 2)

A water dispersion of a granular copolymer 2 was obtained in a same manner as in Example 1-1, except that the amount of St was changed from 27.8 g in Example 1-1 to 35.0 g, the amount of GMA was changed from 17.3 g to 9.7 g, and the amount of additional GMA was changed from 5.2 g in two times to 6.2 g in two times. After slowly cooling the dispersion to room temperature, a part of the dispersion was collected to evaluate the polymerization conversion rate in a same manner as in Example 1-1. As a result, the polymerization conversion rate was confirmed to be substantially 100%. In other words, the mole ratio of GMA relative to the total of St and GMA in the granular copolymer 2 is 32.7 mol%. Also, a mass ratio of the amount of GMA added relative to the amount of monomer at start of polymerization is 0.27. A volume average particle size of the granular copolymer 2 was 245 nm.

[Example 1-3]

(Synthesis of granular copolymer (mother particle) 3)

A water dispersion of a granular copolymer 3 was obtained in a same manner as in Example 1-1, except that the amount of GMA was changed from 17.3 g in Example 1-1 to 19.3 g, and the amount of additional GMA was changed from 5.2 g in two times to 4.1 g in two times. After slowly cooling the dispersion to room temperature, a part of the dispersion was collected to evaluate the polymerization conversion rate in an same manner as in Example 1-1. As a result, the polymerization conversion rate was confirmed to be substantially 100%.

In other words, the mole ratio of GMA relative to the total of St and GMA in the granular copolymer 3 is 41.9 mol%. Also, the mass ratio of the amount of GMA added relative to the amount of monomer at start of polymerization is 0.17. A volume average particle size of the granular copolymer 3 was 234 nm.

[Example 1-4]

(Synthesis of granular copolymer (mother particle) 4)

A water dispersion of a granular copolymer 4 was obtained in a same manner as in Example 1-1, except that the amount of ion-exchanged water was changed from 1305.5 g in Example 1-1 to 1958.3 g, and the amount of V-50 was changed from 0.68 g to 1.02 g. After slowly cooling the dispersion to room temperature, a part of the dispersion was collected to evaluate the polymerization conversion rate in the same manner as in Example 1-1. As a result, the polymerization conversion rate was confirmed to be substantially 100%.

In other words, the mole ratio of GMA relative to the total of St and GMA in the granular copolymer 4 and the mass ratio of the amount of GMA added relative to the amount of monomer at start of polymerization are 42.2 mol% and 0.23, respectively as in Example 1-1. A volume average particle size of the granular copolymer 4 was 212 nm.

[Example 1-5]

(Synthesis of granular copolymer (mother particle) 5)

A water dispersion of a granular copolymer 5 was obtained in a same manner as in Example 1-1, except that St in Example 1-1 was changed to methyl methacrylate (MMA, manufactured by Tokyo Chemical Industry Co., Ltd.). After slowly cooling the dispersion to room temperature, a part of the dispersion was collected to evaluate the polymerization conversion rate in a same manner as in Example 1-1. As a result, the polymerization conversion rate was confirmed to be substantially 100%. In other words, in the granular copolymer 5, the mole ratio of GMA relative to the total of methyl methacrylate and GMA is 41.2 mol%. A mass ratio of an additional GMA relative to an amount of monomer at start of polymerization is 0.23. A volume average particle size of the granular copolymer 5 was 262 nm.

[Example 1-6]

(Synthesis of granular copolymer (mother particle) 6)

A water dispersion of a granular copolymer 6 was obtained in a same manner as in Example 1-1, except that the amount of GMA in Example 1-1 was changed to 6.0 g, the amount of additional GMA was changed from 5.2 g in two times to 4.2 g in two times, and the additional GMA was added after 6 hours from start of polymerization. After slowly cooling the dispersion to room temperature, a part of the dispersion was collected to evaluate the polymerization conversion rate in a same manner as in Example 1-1. As a result, the polymerization conversion rate was confirmed to be substantially 100%. In other words, in the granular copolymer 6, a mole ratio of GMA relative to the total of St and GMA is 27.5 mol%. A mass ratio of an additional GMA relative to an amount of monomer at start of polymerization is 0.27. A volume average particle size of the granular copolymer 2 was 232 nm.

[Example 2-1]

(Synthesis of particle 1)

In a 100-ml round bottom flask, 2.5 wt% water dispersion of 24 g of granular copolymer 1, 3.3 g of ion-exchanged water, 80 mg (0.53 mmol) of mercaptosuccinic acid (MSA, manufactured by FUJIFILM Wako Pure Chemical Corporation), and 230 mg (2.12 mmol) of 3-mercapto-1,2-propanediol (MPD, manufactured by FUJIFILM Wako Pure Chemical Corporation) were weighed out, and triethylamine (manufactured by Kishida Chemical Co., Ltd.) was added thereto to adjust pH to 11.3. While stirring the mixture in the round bottom flask at 200 rpm, the temperature was raised to 70°C, and the state was then kept for 18 hours. Thereby, a dispersion of the particle 1 was obtained. The particle 1 was separated from the dispersion with a centrifugal machine. Further, the operation to redisperse the particle 1 in ion-exchanged water was repeated 8 times to purify the particle 1. Finally, a water dispersion including 1.0 wt% of the particle 1 was prepared and stored.

The storage was performed under light-shielding conditions at 4°C. In Table 1-1, physical properties of the particle are shown.

[Example 2-2]

(Synthesis of particle 2)

A water dispersion of particle 2 was obtained in a same manner as in Example 2-1, except that the granular copolymer 1 in Example 2-1 was changed to a granular copolymer 2. In Table 1-1, physical properties of the particle are

shown.

[Example 2-3]

(Synthesis of particle 3)

A water dispersion of particle 3 was obtained in a same manner as in Example 2-1, except that the granular copolymer 1 in Example 2-1 was changed to a granular copolymer 3. In Table 1-1, physical properties of the particle are shown.

[Example 2-4]

(Synthesis of particle 4)

A water dispersion of particle 4 was obtained in a same manner as in Example 2-1, except that pH=11.3 in Example 2-1 was changed to 10.0. In Table 1-1, physical properties of the particle are shown.

[Example 2-5]

(Synthesis of particle 5)

A water dispersion of particle 5 was obtained in the same manner as in Example 2-1, except that the granular copolymer 1 in Example 2-1 was changed to a granular copolymer 4. In Table 1-1, physical properties of the particle are shown.

[Example 2-6]

(Synthesis of particle 6)

A water dispersion of particle 6 was obtained in a same manner as in Example 2-1, except that 80 mg (0.53 mmol) of mercaptosuccinic acid in Example 2-1 was changed to 78 mg of 2-aminopentanedioic acid (APA: L-glutamic acid, manufactured by FUJIFILM Wako Pure Chemical Corporation). In Table 1-2, physical properties of the particle are shown.

[Example 2-7]

(Synthesis of particle 7)

A water dispersion of particle 7 was obtained in a same manner as in Example 2-1, except that 230 mg (2.12 mmol) of 3-mercapto-1,2-propanediol in Example 2-1 was changed to 193 mg (2.12 mmol) of 2-amino-1,3-propanediol (2APD, manufactured by Tokyo Chemical Industry Co., Ltd.). In Table 1-2, physical properties of the particle are shown.

[Example 2-8]

(Synthesis of particle 8)

A water dispersion of particle 8 was obtained in a same manner as in Example 2-1, except that the granular copolymer 1 in Example 2-1 was changed to a granular copolymer 5. In Table 1-2, physical properties of the particle are shown.

[Example 2-9]

(Synthesis of particle 9)

A water dispersion of particle 9 was obtained in a same manner as in Example 2-1, except that 80 mg (0.53 mmol) of mercaptosuccinic acid in Example 2-1 was changed to 20 mg (0.13 mmol), and 230 mg (2.12 mmol) of 3-mercapto-1,2-propanediol was changed to 273 mg (2.52 mmol). In Table 1-2, physical properties are shown.

[Example 2-10]

(Synthesis of particle 10)

A water dispersion of particle 10 was obtained in a same manner as in Example 2-1, except that the granular copolymer 1 in Example 2-1 was changed to a granular copolymer 6, 80 mg (0.53 mmol) of mercaptosuccinic acid was changed to 480 mg (3.18 mmol), and 230 mg (2.12 mmol) of 3-mercapto-1,2-propanediol was changed to 345 mg (3.18 mmol) of 3-amino-1,2-propanediol (3APD, manufactured by Tokyo Chemical Industry Co., Ltd.). In Table 1-2, physical properties are shown.

[Comparative Example 1-1]

(Synthesis of comparative granular copolymer (mother particle) 1)

A water dispersion of a comparative granular copolymer 1 was obtained in a same manner as in Example 1-1, except that the amount of St was changed from 27.8 g in Example 1-1 to 22.0 g, the amount of GMA was changed from 17.3 g to 28.0 g, and the amount of additional GMA was changed to 5.8 g in one time only after 2 hours from the start of polymerization. After slowly cooling the dispersion to room temperature, a part of the dispersion was collected to evaluate the polymerization conversion rate in a same manner as in Example 1-1. As a result, the polymerization conversion rate was confirmed to be substantially 100%. In other words, a mole ratio of GMA relative to the total of St and GMA in the comparative granular copolymer 1 is 53.0 mol%. Also, a mass ratio of the amount of GMA added relative to the amount of monomer at start of polymerization is 0.11. The volume average particle size of the comparative granular copolymer 1 was 238 nm.

[Comparative Example 1-2]

(Synthesis of comparative granular copolymer (mother particle) 2)

A water dispersion of a comparative granular copolymer 2 was obtained in a same manner as in Example 1-1, except that the amount of GMA was changed from 17.3 g in Example 1-1 to 21.8 g, and the amount of additional GMA and a timing of addition were changed to 5.8 g in one time only after 2 hours from the start of polymerization. After slowly cooling the dispersion to room temperature, a part of the dispersion was collected to evaluate the polymerization conversion rate in a same manner as in Example 1-1. As a result, the polymerization conversion rate was confirmed to be substantially 100%. In other words, although the mole ratio of GMA relative to the total of St and GMA in the comparative granular copolymer 2 is 42.2 mol% as in Example 1-1, a mass ratio of the amount of GMA added relative to the amount of monomer at the start of polymerization is 0.12. A volume average particle size of the comparative granular copolymer 2 was 239 nm.

[Comparative Example 1-3]

(Synthesis of comparative granular copolymer (mother particle) 3)

A water dispersion of a comparative granular copolymer 3 was obtained in a same manner as in Example 1-1, except that the amount of GMA was changed from 17.3 g in Example 1-1 to 13.4 g, the amount of additional GMA was changed to 4.8 g in one time, and a timing of addition was changed to three times, i.e. after 2 hours from start of polymerization, 30 minutes after a first addition, and 30 minutes after a second addition. After slowly cooling the dispersion to room temperature, a part of the dispersion was collected to evaluate the polymerization conversion rate in a same manner as in Example 1-1. As a result, the polymerization conversion rate was confirmed to be substantially 100%. In other words, although a mole ratio of GMA relative to the total of St and GMA in the comparative granular copolymer 3 is 42.2 mol% as in Example 1-1, a mass ratio of the amount of GMA added relative to the amount of monomer at the start of polymerization is 0.34. A volume average particle size of the comparative granular copolymer 3 was 243 nm.

[Comparative Example 2-1]

(Synthesis of comparative particle 1)

A water dispersion of comparative particle 1 was obtained in a same manner as in Example 2-1, except that the granular copolymer 1 in Example 2-1 was changed to the comparative granular copolymer 1. The physical properties of the particle are shown in Table 2.

[Comparative Example 2-2]

(Synthesis of comparative particle 2)

A water dispersion of comparative particle 2 was obtained in a same manner as in Example 2-1, except that the granular copolymer 1 in Example 2-1 was changed to the comparative granular copolymer 2. The physical properties of the particle are shown in Table 2.

[Comparative Example 2-3]

(Synthesis of comparative particle 3)

A water dispersion of comparative particle 3 was obtained in a same manner as in Comparative Example 2-2, except that 80 mg (0.53 mmol) of mercaptosuccinic acid was changed to 20 mg (0.13 mmol), and 230 mg (2.12 mmol) of 3-mercapto-1,2-propanediol was in Comparative Example 2-2 changed to 273 mg (2.52 mg). The physical properties of the particle are shown in Table 2.

[Comparative Example 2-4]

(Synthesis of comparative particle 4)

A water dispersion of comparative particle 4 was obtained in a same manner as in Example 2-1, except that the granular copolymer 1 in Example 2-1 was changed to the comparative granular copolymer 3. The physical properties of the particle are shown in Table 2.

[Evaluation 1] Evaluation of non-specific adsorption to particle

In a phosphate buffer solution (containing 0.01% of Tween20), each of particles 1 to 10 and comparative particles 1 to 4 was dispersed to a content of 0.1 wt%, so that a dispersion (liquid P) was prepared. Subsequently, to each 50 μL of the dispersion, 55 μL of a specimen diluent (liquid Q) including a normal human specimen (serum specimen, 5 μL) and a phosphate buffer solution (50 μL) was added and stirred to prepare a mixture, which was immediately subjected to measurement of absorbance at a wavelength of 572 nm. In the measurement of absorbance, a spectrophotometer "Biospectrometer" manufactured by Eppendorf SE was used. After that, each of the mixtures was left standing still at 37°C for 5 minutes, and then subjected to measurement of absorbance at a wavelength of 572 nm again to calculate the value of variation in absorbance $\Delta ABS \times 10000$. The results are summarized in Table 1-1, Table 1-2, and Table 2. It is presumed that occurrences of non-specific adsorption increase in proportion to the value. In a case of using the particle for latex agglutination method in specimen test, a concern is that a normal specimen may be determined as false positive. In addition, the resulting value of variation $\Delta ABS \times 10000$ was ranked based on the following criteria, in consideration of noise risk in detection of a target substance at a low concentration by latex agglutination method.

A: less than +50
B: 50 or more and less than 100
C: 100 or more and less than 500
D: 500 or more

From Table 1-1 and Table 1-2, it can be seen that particles 1 to 10, which have a composition ratio of element C to element O in a particle surface layer of 2.1 or more and 3.3 or less, are excellent in ability to suppress non-specific adsorption.

On the other hand, it can be seen that comparative particles 2 and 3, which have a composition ratio of element C to element O of more than 3.3, have a large non-specific adsorption. Although the comparative particles 2 and 3 have a GMA ratio in mother particle below a certain level in order to achieve long-term storage as in Examples, a mother particle synthesized with a ratio of amount of GMA added of less than 16 mass% is used in the comparative particles 2 and 3. As a result, in comparison with the particle of the present invention, a less amount of GMA in the surface layer of mother particle cannot be compensated, so that it is expected that a styrene ratio in a surface layer of mother particle is high. Even in the particle after a hydroxy group and a carboxy group are added, styrene in the surface layer of mother particle is present in the surface layer without change. As a result, the composition ratio of element C to element O in the particle surface layer is high, so that it is expected the comparative particles 2 and 3 have greater non-specific adsorption.

Also from no detection of specific endothermic peak (derived from polystyrene) in DSC curve, it is expected that the comparative particles 2 and 3 have no high concentration area of polystyrene in the particle, and polystyrene is not firmly enclosed in the particle in comparison to the particle of the present invention.

Comparative particle 1 also have no detection of specific endothermic peak in DSC curve, having good non-specific adhesion on the other hand. It is presumed that because comparative particle 1 has higher GMA ratio than a range in the present invention, with less amount of polystyrene in the original particle composition, the behavior derived from polystyrene is hardly observed.

[Evaluation 2] Preparation of test particle by antibody sensitization to particle, and evaluation of antibody sensitization rate.

(Preparation of test particle)

Regarding each of particles 1 to 10 and comparative particles 1 to 4, 180 μL of 1.7 wt% water suspension was served in a 1.5-mL microtube, and 90 μL of 5.0 wt% aqueous solution of 1-(3-dimethylaminopropyl)-3-ethylcarbodiimide hydrochloride and 90 μL of 5.0 wt% aqueous solution of N-hydroxysulfosuccinimide sodium were added therein to make a mixture. The mixture was stirred at room temperature for 30 minutes, so that a dispersion of particles having an activated carboxy group (activated particle dispersion) was obtained.

After centrifugal washing, 270 μL of phosphate buffer solution-normal saline solution having a pH of 7.2 (hereinafter, referred to as PBS) was added, and the particles with carboxy group activated were dispersed by ultrasonic waves.

Thereinto, 5 μL of dispersion of 15.0 mg/mL of clone C5 (manufactured by Funakoshi Co., Ltd.) of monoclonal mouse anti-human C-reactive protein (hereinafter, referred to as CRP antibody) was added and stirred at room temperature for 3 hours, so that a test particle was obtained by antibody sensitization to the particle. Each of the test particles was subjected to centrifugal washing and then 1 mL of PBS were added thereto to make a mixture, and the mixture was stored in a dispersed state.

As a result of evaluation of the specific gravity of the test particle of particle 1, the particle specific gravity was 1.079, while the particle specific gravity before antibody sensitization was 1.082. From the above, it can be determined that the actual particle specific gravity of the test particle as component of a test reagent is equivalent to the particle specific gravity before sensitization.

(Evaluation of antibody sensitization rate of test particle)

Next, according to the method for measuring antibody sensitization rate of test particle described above, the antibody sensitization rate (%) was determined.

The results are summarized in Table 1-1, Table 1-2, and Table 2.

From Table 1-1 and Table 1-2, it can be seen that any of the particles 1 to 10, which have a composition ratio of element C to element O in the particle surface layer of 2.1 or more and 3.3 or less, exhibits a sufficient antibody sensitization rate. It is presumed that particularly in the case where the zeta potential is lower than -10 mV (large absolute value), the particle surface layer is expected to have many carboxy groups, resulting in a particularly high antibody sensitization rate.

In particular, among comparative particles, the comparative particle 3, which has a composition ratio of element C to element O of more than 3.3 and a zeta potential of higher than -10 mV (small absolute value), has a poor antibody sensitization rate. The comparative particle 3 is a particle made from a mother particle synthesized at a ratio of an amount of additional GMA of less than 16 mass% as described in [Evaluation 1], and an amount of GMA in the surface layer of mother particle is small, so that imparting sufficient amount of carboxy groups is inherently difficult. Further, the comparative particle 3 uses less amount of mercaptosuccinic acid, having less amount of carboxy group as predicted from a small absolute value of a zeta potential, so that it is presumed that bonding to sufficient amount of antibody is not achieved.

[Evaluation 3] Evaluation of latex agglutination sensitivity and evaluation of non-specific adsorption of test particle

(Evaluation of standard serum)

A standard serum for CRP was diluted with PBS to a concentration of 0.15 mg/dL to make a CRP specimen solution. The CRP specimen solution in an amount of 1 μL and a buffer solution (PBS containing 0.01% Tween20) in an amount of 50 μL were mixed to prepare a mixture (hereinafter, referred to as R1+), which was kept warm at 37°C. As a control, normal saline solution in an amount of 1 μL and a buffer solution (PBS containing 0.01% Tween20) in an amount of 50 μL were mixed to prepare a mixture (hereinafter, referred to as R1-), which was similarly kept warm at 37°C.

Next, the dispersion of each of the test particles sufficiently dispersed by ultrasonic waves again before use (particle concentration: 0.1 wt%, referred to as R2) in amount of 50 μL was mixed with R1+ or R1-. The mixture (volume: 101 μL) immediately after stirring was subjected to measurement of absorbance at a wavelength of 572 nm. A spectrophotometer "Biospectrometer" manufactured by Eppendorf SE was used in the measurement of absorbance. The mixture

was left standing still at 37°C for 5 minutes and then subjected to measurement of absorbance at a wavelength of 572 nm again to calculate a value of variation in absorbance ΔABS×10000. Results are summarized in Table 1-1, Table 1-2, and Table 2.

A large value of R- (reactivity with normal saline solution) means that occurrence of agglutination caused by non-specific adsorption or osmotic flocculation in the test particle. In that case, use of particle for latex agglutination method in specimen test causes a concern that a normal specimen may be subjected to false positive due to noise. However, it has been confirmed that no such agglutination occurred in all the particles evaluated in the present Examples.

Larger the R+ value (reactivity with 0.15 mg/dl human CRP) of a test particle for use of particle for latex agglutination method in specimen test is, more sensitive detection of a target substance is expected.

The resulting R+ values were ranked based on the following criteria.

A: 1500 or more
B: 1200 or more and less than 1500
C: 1000 or more and less than 1200
D: less than 1000

From Table 1-1 and Table 1-2, it can be seen that the test particles of particles 1 to 10, which have a composition ratio of element C to element O of 2.1 or more and 3.3 or less, can detect a human CRP at a concentration (0.15 mg/dl) lower than the standard value with high sensitivity by latex agglutination method. Detection sensitivity is excellent particularly in a case where the volume average particle size is more than 280 nm, in a case where the zeta potential is lower than -10 mV (large absolute value) with sufficient antibody sensitization rate, and in a case where a granular copolymer of styrene and glycidyl methacrylate is used as mother particle.

On the other hand, the comparative particle 4, which has a composition ratio of element C to element O of less than 2.1, has poor sensitivity in the latex agglutination method. The comparative particle 4 is a particle made from mother particle synthesized with a ratio of amount of additional GMA of more than 30 mass%. As a result, an amount of GMA in a mother particle surface layer is large, so that sufficient hydroxy groups and carboxy groups are obtained with a high antibody sensitization rate. On the other hand, due to the extremely high hydrophilicity, agglutination of particles hardly occurs, resulting in lowered detection sensitivity based on the principle of latex agglutination method. It is also expected that the low antibody sensitization amount of the comparative particle 3 described in [Evaluation 2] resulted in insufficient detection sensitivity.

[Evaluation 4]

(Evaluation of latex agglutination sensitivity after test particle dispersion is stored standing still)

Each of the test particles prepared in [Evaluation 2] was dispersed at a particle concentration of 0.1 wt% in 10 mM HEPES solution having a pH 7.9, with 0.01% Tween20 for use. Each of the dispersions in an amount of 1 mL was fractionated into a 1.5 mL microtube. The dispersion liquid was stored standing still at 4°C for 1 week, and then sedimentation of the particle was subjected to visual test. On this occasion, 50 μL of dispersion was carefully collected from a position at a depth of 3 mm from the liquid surface of the dispersion. Using the liquid, the evaluation of the latex agglutination sensitivity to the human CRP (measurement of R+) was performed in a same manner as in [Evaluation 3]. The results are summarized in Table 1-1, Table 1-2, and Table 2.

The change rate (%) of R+ value was calculated by subtracting a value of ΔABS×10000 obtained in [Evaluation 3] (Δ(1)) from the value of ΔABS×10000 obtained in [Evaluation 4] (Δ(2)), and then dividing the difference by (Δ(1)). The resulting change rate of R+ value was ranked based on the following criteria.

A: -5% or more
B: -10% or more and less than -5%
C: -15% or more and less than -10%
D: less than -15%

As shown in Table 1-1 and Table 1-2, the test particles of particles 1 to 10, which have a specific gravity of particle of 1.10 or less and a composition ratio of element C to element O in a particle surface of 2.1 or more and 3.3 or less, caused no particle sedimentation identified by visual test even after being left standing still for 1 week. Also, the dispersion collected from an upper part of dispersion after static storage caused no large difference in latex agglutination sensitivity in [Evaluation 3] as evaluation immediately after ultrasonic dispersion. It is presumed that in particular, the particle having a volume average particle size of 350 nm or less is excellent particularly in dispersion stability, because due to a particle size, a natural sedimentation rate is slower in comparison with the particles having a size of 350 nm or more as a matter of

course.

On the other hand, the dispersion of the comparison particle 1, which has a particle specific gravity of more than 1.10, had a transparent liquid phase identified near the liquid surface, so that progress of particle sedimentation was expected. Also, the dispersion collected from an upper part of dispersion after static storage had distinctly lowered latex agglutination sensitivity. Also, among the comparative particles having a particle specific gravity of less than 1.10, the comparative particles 2 and 3, which has a composition ratio of element C to element O of 3.3 or more had a slight amount of transparent liquid phase identified by visual test. Due to insufficient hydrophilicity of the particle surface layer, it is presumed that sedimentation was accelerated to some extent. Consequently, the latex agglutination sensitivity of the dispersion collected after static storage was greatly lowered.

[Table 1-1]

| | | | Example | | | | |
|---|---|---|---|---|---|---|---|
| | | | Particle 1 | Particle 2 | Particle 3 | Particle 4 | Particle 5 |
| Granular copolymer (Mother particle) | Number of copolymer of particle | | 1 | 2 | 3 | 1 | 4 |
| | Monomer from which structural unit A and structural unit B are derived | | GMA | GMA | GMA | GMA | GMA |
| | Monomer from which structural unit C is derived | | St | St | St | St | St |
| | Number of times of addition of additional GMA | | 2 times | 2 times | 2 times | 2 times | 2 times |
| | Mass ratio of amount of GMA added relative to amount of monomer at start of polymerization | | 0.23 | 0.29 | 0.17 | 0.23 | 0.23 |
| | Volume average particle size (nm) | | 240 | 245 | 236 | 240 | 212 |
| Particle | Total amount of structural unit A and structural unit B / Total amount of structural unit A, structural unit B and structural unit C (mol%) | | 42.2 | 32.7 | 41.9 | 42.2 | 42.2 |
| | Origin of structural unit A (reagent for imparting hydroxy group) | | MPD | MPD | MPD | MPD | MPD |
| | Origin of structural unit B (reagent for imparting carboxyl group) | | MSA | MSA | MSA | MSA | MSA |
| | Volume average particle size (nm) | | 297 | 281 | 292 | 283 | 262 |
| | Specific gravity | | 1.082 | 1.062 | 1.087 | 1.082 | 1.083 |
| | Composition ratio of element C to element O quantified by XPS | | 2.67 | 2.16 | 2.92 | 2.49 | 2.47 |
| | Presence or absence of intersection point A and endothermic peak in specific temperature range of DSC curve | | present | present | present | present | present |
| | Zeta potential (mV) | | -23.1 | -24.5 | -20.9 | -16.7 | -21.1 |
| | [Evaluation 1] Non-specific adsorption | Reactivity with normal human specimen | 40 | 30 | 80 | 90 | 20 |
| | | Rank | A | A | B | B | A |

(continued)

| | | | Example | | | | |
|---|---|---|---|---|---|---|---|
| | | | Particle 1 | Particle 2 | Particle 3 | Particle 4 | Particle 5 |
| Test particle | [Evaluation 2] Antibody sensitization rate | Anti-human CRP antibody sensitization rate (%) | 93 | 79 | 96 | 90 | 89 |
| | [Evaluation 3] Latex agglutination sensitivity | R-(Reactivity with normal saline solution) (ΔABS×10000) | 10 | -30 | 0 | 10 | 10 |
| | | (Δ(1)) R+(Reactivity with 0.15 mg/dl of human CRP) (ΔABS×10000) | 1640 | 1850 | 1780 | 1590 | 1240 |
| | | Rank | A | A | A | A | B |
| | [Evaluation 4] Latex agglutination sensitivity after static storage | (Δ(2)) R+(Reactivity with 0.15 mg/dl of human CRP) (ΔABS×10000) | 1680 | 1870 | 1710 | 1600 | 1260 |
| | | Change rate of value of R+ (%) | 2.4% | 1.1% | -3.9% | 0.6% | 1.6% |
| | | Rank | A | A | A | A | A |
| | | Visual observation after static storage | No change was observed. | | | | |

[Table 1-2]

| | | Example | | | | |
|---|---|---|---|---|---|---|
| | | Particle 6 | Particle 7 | Particle 8 | Particle 9 | Particle 10 |
| Granular copolymer (Mother particle) | Number of copolymer of particle | 1 | 1 | 5 | 1 | 6 |
| | Monomer from which structural unit A and structural unit B are derived | GMA | GMA | GMA | GMA | GMA |
| | Monomer from which structural unit C is derived | St | St | MMA | St | St |
| | Number of times of addition of additional GMA | 2 times | 2 times | 2 times | 2 times | 2 times |
| | Mass ratio of amount of GMA added relative to amount of monomer at start of polymerization | 0.23 | 0.23 | 0.23 | 0.23 | 0.25 |
| | Volume average particle size (nm) | 240 | 240 | 262 | 240 | 232 |

(continued)

| | | | Example | | | | |
|---|---|---|---|---|---|---|---|
| | | | Particle 6 | Particle 7 | Particle 8 | Particle 9 | Particle 10 |
| Particle | Total amount of structural unit A and structural unit B / Total amount of structural unit A, structural unit B and structural unit C (mol%) | | 42.2 | 42.2 | 41.2 | 42.2 | 27.5 |
| | Origin of structural unit A (reagent for imparting hydroxy group) | | MPD | 2APD | MPD | MPD | 3APD |
| | Origin of structural unit B (reagent for imparting carboxyl group) | | APA | MSA | MSA | MSA | MSA |
| | Volume average particle size (nm) | | 310 | 303 | 355 | 294 | 289 |
| | Specific gravity | | 1.086 | 1.075 | 1.095 | 1.085 | 1.041 |
| | Composition ratio of element C to element O quantified by XP S | | 3.12 | 2.81 | 2.17 | 2.2 | 2.24 |
| | Presence or absence of intersection point A and endothermic peak in specific temperature range of DSC curve | | present | present | absent | present | present |
| | Zeta potential (mV) | | -20.5 | -19.2 | -22.2 | -9.6 | -34.1 |
| | [Evaluation 1] Non-specific adsorption | Reactivity with normal human specimen | 190 | 0 | 50 | -20 | 0 |
| | | Rank | C | A | B | A | A |
| Test particle | [Evaluation 2] Antibody sensitization rate | Anti-human CRP antibody sensitization rate (%) | 82 | 86 | 99 | 71 | 79 |
| | [Evaluation 3] Latex agglutination sensitivity | R-(Reactivity with normal saline solution) (ΔABS×10000) | 0 | 10 | 20 | -10 | -30 |
| | | (Δ(1)) R+(Reactivity with 0.15 mg/dl of human CRP) (ΔABS×10000) | 1310 | 1540 | 1160 | 1080 | 2040 |
| | | Rank | B | A | C | C | A |
| | [Evaluation 4] Latex agglutination sensitivity after static storage | (Δ(2)) R+(Reactivity with 0.15 mg/dl of human CRP) (ΔABS×10000) | 1280 | 1520 | 1050 | 1110 | 2030 |
| | | Change rate of value of R+ (%) | -2.3% | -1.3% | -9.5% | 2.8% | -0.5% |
| | | Rank | A | A | B | A | A |
| | | Visual observation after static storage | No change was observed. | | | | |

[Table 2]

| | | | Comparative Example | | | |
|---|---|---|---|---|---|---|
| | | | Comparative particle 1 | Comparative particle 2 | Comparative particle 3 | Comparative particle 4 |
| Granular copolymer (Mother particle) | Number of copolymer of comparative particle | | 1 | 2 | 2 | 3 |
| | Monomer from which structural unit A and structural unit B are derived | | GMA | GMA | GMA | GMA |
| | Monomer from which structural unit C is derived | | St | St | St | St |
| | Number of times of addition of additional GMA | | 1 time | 1 time | 1 time | 3 times |
| | Mass ratio of amount of GMA added relative to amount of monomer at start of polymerization | | 0.12 | 0.12 | 0.12 | 0.35 |
| | Volume average particle size (mn) | | 238 | 239 | 239 | 243 |
| Particle | Total amount of structural unit A and structural unit B / Total amount of structural unit A, structural unit B and structural unit C (mol%) | | 53.0 | 42.1 | 42.1 | 42.3 |
| | Origin of structural unit A (reagent for imparting hydroxy group) | | MPD | MPD | MPD | MPD |
| | Origin of structural unit B (reagent for imparting carboxyl group) | | MSA | MSA | MSA | MSA |
| | Volume average particle size (mn) | | 298 | 283 | 278 | 311 |
| | Specific gravity | | 1.124 | 1.08 | 1.089 | 1.085 |
| | Composition ratio of element C to element O quantified by XPS | | 2.9 | 3.47 | 3.39 | 2.01 |
| | Presence or absence of intersection point A and endothermic peak in specific temperature range of DSC curve | | absent | absent | absent | present |
| | Zeta potential (mV) | | -23.7 | -20.8 | -8 | -21.4 |
| | [Evaluation 1] Non-specific adsorption | Reactivity with normal human specimen | 90 | 3160 | 2850 | 30 |
| | | Rank | B | D | D | A |

(continued)

| | | | Comparative Example | | | |
|---|---|---|---|---|---|---|
| | | | Comparative particle 1 | Comparative particle 2 | Comparative particle 3 | Comparative particle 4 |
| Test particle | [Evaluation 2] Antibody sensitization rate | Anti-human CRP antibody sensitization rate (%) | 95 | 80 | 56 | 93 |
| | [Evaluation 3] Latex agglutination sensitivity | R-(Reactivity with normal saline solution) (ΔABS×10000) | 20 | -10 | 10 | 90 |
| | | (Δ(1)) R+(Reactivity with 0.15 mg/dl of human CRP) (ΔABS×10000) | 1420 | 1350 | 460 | 520 |
| | | Rank | B | B | D | D |
| | [Evaluation 4] Latex agglutination sensitivity after static storage | (Δ(2)) R+(Reactivity with 0.15 mg/dl of human CRP) (ΔABS×10000) | 950 | 1180 | 400 | 560 |
| | | Change rate of value of R+ (%) | -33.1% | -12.6% | -13.0% | 7.7% |
| | | Rank | D | D | D | A |
| | | Visual observation after static storage | Transparent liquid phase was identified near liquid surface. | Transparent liquid phase was slightly identified near liquid surface. | | No change was observed. |

According to the present invention, a particle for use in latex agglutination method, having a reactive functional group for chemically bonding to a ligand, with small non-specific adsorption, excellent in stability during static storage, and a production method thereof can be provided. Further, a test particle including a chemically bonded ligand, a reagent and kit for in vitro diagnosis containing the particle, and a method for detecting a target substance can be provided.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

## Claims

**1.** A test particle comprising:

a ligand, and
a particle chemically bonded to the ligand, wherein the particle has a polymer including a structural unit A represented by the following formula (1) and a structural unit B represented by the following formula (2),
the particle has a specific gravity in a range of 1.00 or more and 1.10 or less, and
the particle has a composition ratio of element C to element O quantified by XPS measurement in a range of 2.1 or more and 3.3 or less:

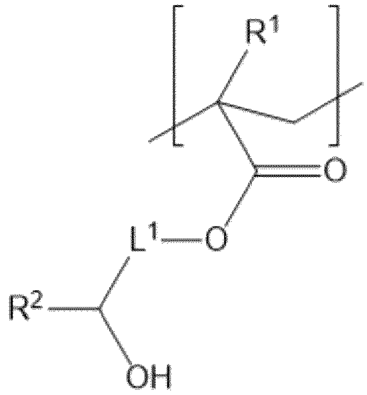

(1)

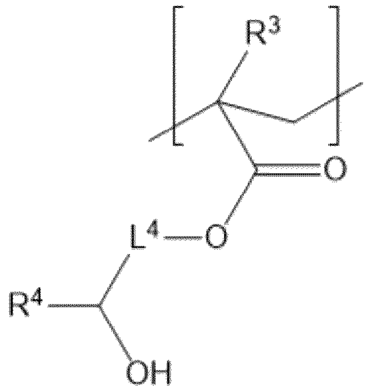

(2)

wherein, in the formula (1), $R^1$ represents a methyl group or a hydrogen atom, $L^1$ represents an alkylene group having 1 or more and 4 or less carbon atoms, and $R^2$ is a group including a sulfide group or a secondary amine and a hydroxy group; and wherein, in the formula (2), $R^3$ represents a methyl group or a hydrogen atom, $L^4$ represents an alkylene group having 1 or more and 4 or less carbon atoms, and $R^4$ is a group including a sulfide group or a secondary amine and a carboxy group.

**2.** The test particle according to claim 1, wherein the particle further comprises a structural unit C represented by the following formula (3) or the following formula (4),

wherein a total amount of the structural unit A and the structural unit B in a total amount of the structural unit A, the structural unit B and the structural unit C is 5 mol% or more and 43 mol% or less:

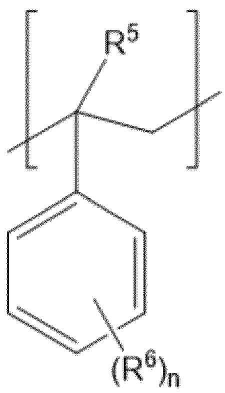

(3)

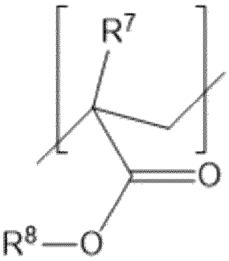

(4)

wherein, in the formula (3), $R^5$ represents a methyl group or a hydrogen atom, $R^6$ represents a straight-chain or branched alkyl group having 1 or more and 9 or less carbon atoms or a hydrogen atom, and n represents an integer of 1 or more and 5 or less; and wherein, in the formula (4), $R^7$ represents a methyl group or a hydrogen atom, and $R^8$ represents a straight-chain or branched alkyl group having 1 or more and 12 or less carbon atoms.

3. The test particle according to claim 1 or 2, wherein the particle has a zeta potential of -50 mV or more and -10 mV or less.

4. The test particle according to claim 2, wherein the structural unit C is at least one selected from the group consisting of styrenes.

5. The test particle according to claim 2 or 4, wherein an amount of the structural unit C in the total amount of the structural unit A, the structural unit B and the structural unit C is 57 mol% or more and 95 mol% or less.

6. The test particle according to any one of claims 1 to 5, wherein when on a DSC curve obtained in a second temperature rise in differential scanning calorimetry (DSC) measurement of the particle, a straight line passing through a point on the DSC curve at a temperature of 80°C and a point on the DSC curve at a temperature of 100°C is drawn, and an intersection point between the straight line and the DSC curve in a temperature region of 105°C or more and 140°C or less is defined as intersection point A, the DSC curve has an endothermic peak in a temperature region between a temperature At of the intersection point A and 100°C.

7. The test particle according to any one of claims 1 to 6, wherein the structural unit A contains a structure represented by the following formula (5):

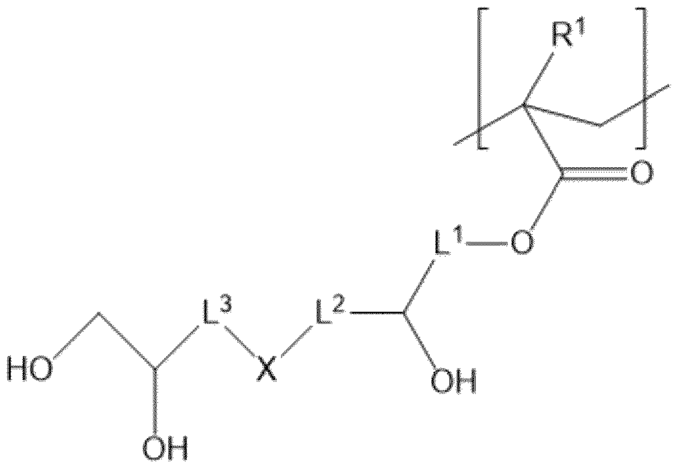

(5)

wherein $R^1$ represents a methyl group or a hydrogen atom, $L^1$ represents an alkylene group having 1 or more and 4 or less carbon atoms, $L^2$ and $L^3$ each independently represent any one of a single bond and an alkylene group having 1 to 3 carbon atoms, and X represents S or NH.

8. The test particle according to any one of claims 1 to 7, wherein the structural unit B contains a structure represented by the following formula (6):

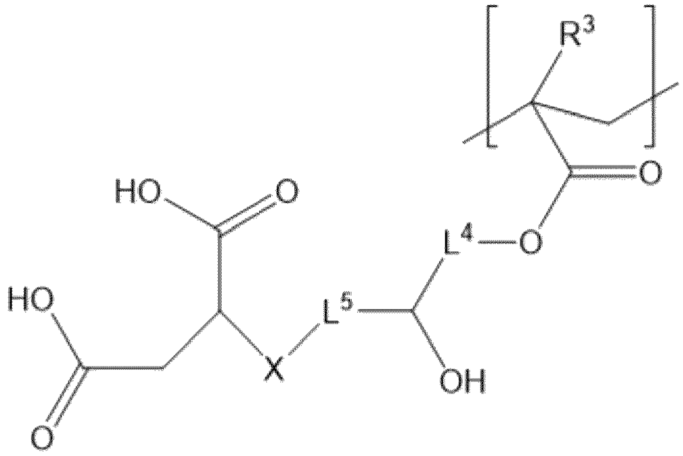

(6)

wherein $R^3$ represents a methyl group or a hydrogen atom, $L^4$ represents an alkylene group having 1 or more and 4 or less carbon atoms, $L^5$ represents any one of a single bond and an alkylene group having 1 or more and 3 or less carbon atoms, and X represents S or NH.

**9.** The test particle according to claim 2, 4, or 5, wherein the structural unit C is styrene.

**10.** The test particle according to any one of claims 1 to 9, wherein the particle has a volume average particle size in water dispersion of 280 nm or more and 400 nm or less.

**11.** The test particle according to any one of claims 1 to 10, wherein the ligand is an antibody or an antigen.

**12.** A reagent for detecting a target substance in a specimen by in vitro diagnosis, comprising a test particle according to any one of claims 1 to 11 and dispersion medium.

**13.** The reagent according to claim 12, for use in detecting a target substance in a specimen by agglutination method.

**14.** A kit for use in detecting a target substance in a specimen by in vitro diagnosis, comprising at least the reagent according to claim 12 or 13.

**15.** A method for detecting a target substance in a specimen by agglutination reaction, comprising:
mixing a test particle according to any one of claims 1 to 11 with a specimen possibly containing a target substance.

**16.** A method for producing a particle, comprising the following steps:

step 1: mixing a compound represented by the following formula (7), a compound represented by the following formula (8), water and a radical polymerization initiator to initiate polymerization;
step 2: further adding a compound represented by the formula (7) to a reaction system after the step 1;
wherein a ratio of a mass of the compound represented by the formula (7) added in the step 2 relative to a sum of a mass of the compound represented by the formula (7) and a mass of the compound represented by the formula (8) mixed in the step 1 is 0.16 or more and 0.30 or less:

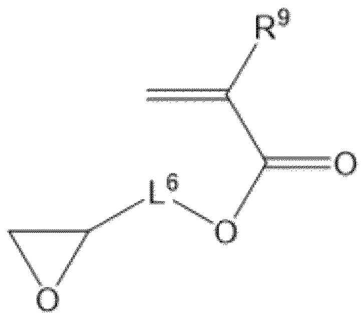

(7)

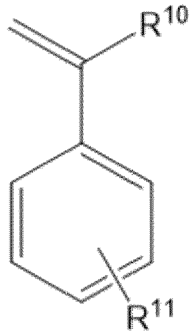

(8)

wherein, in the formula (7), $R^9$ represents a methyl group or a hydrogen atom, and $L^6$ represents an alkylene group having 1 or more and 4 or less carbon atoms; and wherein, in the formula (8), $R^{10}$ represents a methyl group or a hydrogen atom, and $R^{11}$ represents a straight-chain or branched alkyl group having 1 or more and 9 or less carbon atoms or a hydrogen atom.

FIG. 1

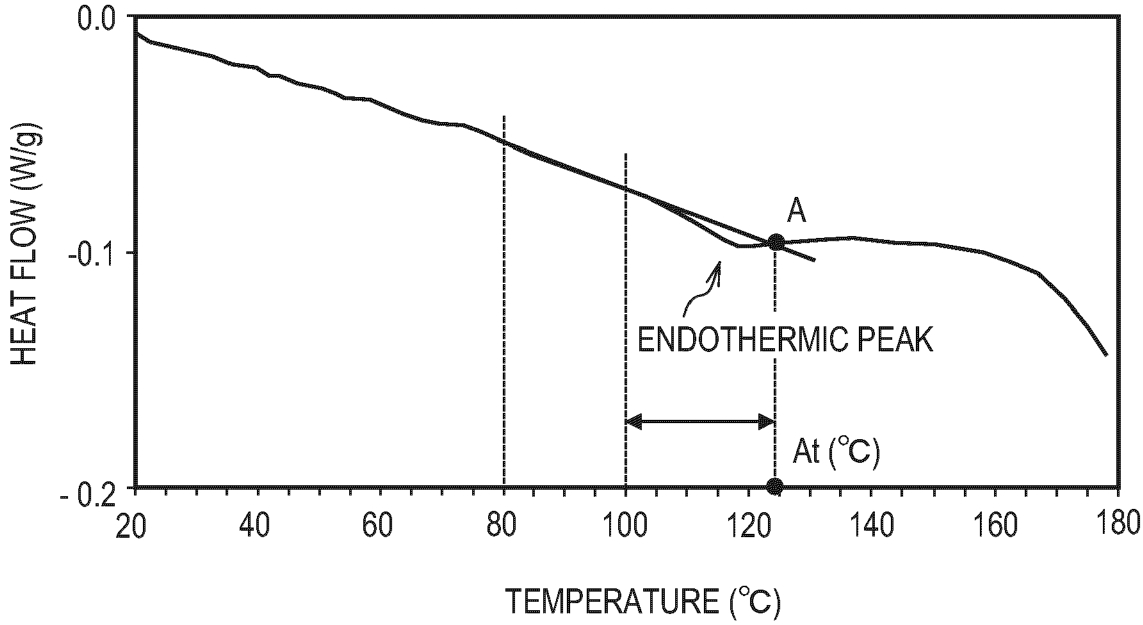
0.0
-0.1
- 0.2
HEAT FLOW (W/g)
A
ENDOTHERMIC PEAK
At (°C)
20
40
60
80
100
120
140
160
180
TEMPERATURE (°C)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2000351814 A
- JP 2006071297 A